# EUROPEAN PATENT SPECIFICATION

(11) **EP 1 084 634 B1**
(45) Date of publication and mention of the grant of the patent: **06.11.2002**
(21) Application number: 98924329.0
(22) Date of filing: 11.06.1998
(51) Int. Cl.: A41F 1/04, A61F 5/03

(54) **BAND FOR CLOSING CORSETS**
BAND ZUM VERSCHLIESSEN EINES KORSETTS
BANDE POUR FERMETURE DE CORSETS

(43) Date of publication of application: 21.03.2001
(73) Proprietor: Prim, S.A., 28938 Mostoles (ES)
(72) Inventor: MEIJIDE GARCIA, Jose Luis, E-28938 Mostoles (ES)
(74) Representative: Perez Bonal, Bernardo
(86) International application number: PCT/ES98/00168
(87) International publication number: WO 99/063848

(56) References cited:
- US-A- 1 665 502
- US-A- 1 736 128
- US-A- 2 017 679
- US-A- 2 066 536
- US-A- 2 406 528
- US-A- 3 091 774

## Description

### OBJECT OF THE INVENTION

The invention here disclosed relates to a band with eyelet and housing for stays, from among supporting bands in closing means for corsets acting as stay protection sheaths in corsets and similar elements.

This invention is characterised by a special construction of the band, which is provided with a resurfacing of one of its sides, incorporating a number of lace segments which in its free areas defines eyelets along the band.

### BACKGROUND OF THE INVENTION

Protection bands which act as stay sheaths are widely known and used in female clothing in general, and particularly in orthopaedic corsets.

An example of the above is the sturdy woven band of Utility Model no. 013712, "Stay protector sheath in orthopaedic corsets and the like" provided with a perimetral seam which prevents the stay ends from sliding out of it.

Furthermore, clothing item edge eyelets are unnecessarily thick due to the conventional construction of the eyelet, which is riveted to and salient from the rest of the item, unnecessarily increasing its perimeter as it is rolled around the user's body, and therefore also increasing the latter's perimeter.

There are some methods used for tightening and closing an elastic band such as the one described in the US 1736128 patent which uses eyes for regulating the width of the item and loops for tightening to the anatomy of the wearer, we propose to tight and to close the item with only one movement and using the same eyelets, being built in a way that to allow to distribute the efforts in the item.

### DESCRIPTION OF THE INVENTION

The invention object of the present memory relates to a band with eyelets and housing for stays, preferably from among woven bands, even when this construction is not necessarily the preferred one, bands which house the stays and encase them for the purposes of conventional uses, both for corsets and for similar elements in clothing items.

The invention is characterised by a particular construction of the band which is provided in one of its sides with a number of segments which are resurfaced and which house a number of corresponding segments of laces of equal lengths, in any construction of the latter, albeit observing tension resistance properties suitable for their purpose.

The band is preferably of double construction, as well as the sewn edges, so that the stay may be housed inside it, and reinforced on its sides with the consecutive seams of the lace segments.

The aforementioned lace defines in its free areas segments which extend outwards markedly, describing a half-wave crest.

These crest projections define eyelets placed along the band with the equidistance maintained by the corresponding segments which are joined to said band.

These laterally and continuously placed eyelets are used to insert further conventional laces for tightening and closing in corsets, using all or some of the eyelets as desired.

In any case the reinforcements on the band sides secure the eyelets along the segment in which they are in contact with the band, as opposed to the conventional construction in which each eyelet is in a weakened area of the support with the ensuing risk of tearing.

In the case of constructing a corset it is sufficient to add to this arrangement of bands with eyelets and adjustment and closing laces portions of a complementary fabric to which they are sowed in order to obtain the aforementioned item.

### DESCRIPTION OF THE DRAWINGS

Further characteristics of the present invention will become apparent in view of the accompanying drawings where, for purposes of illustration only and in no way meant to define the limits of the invention, the following is shown:

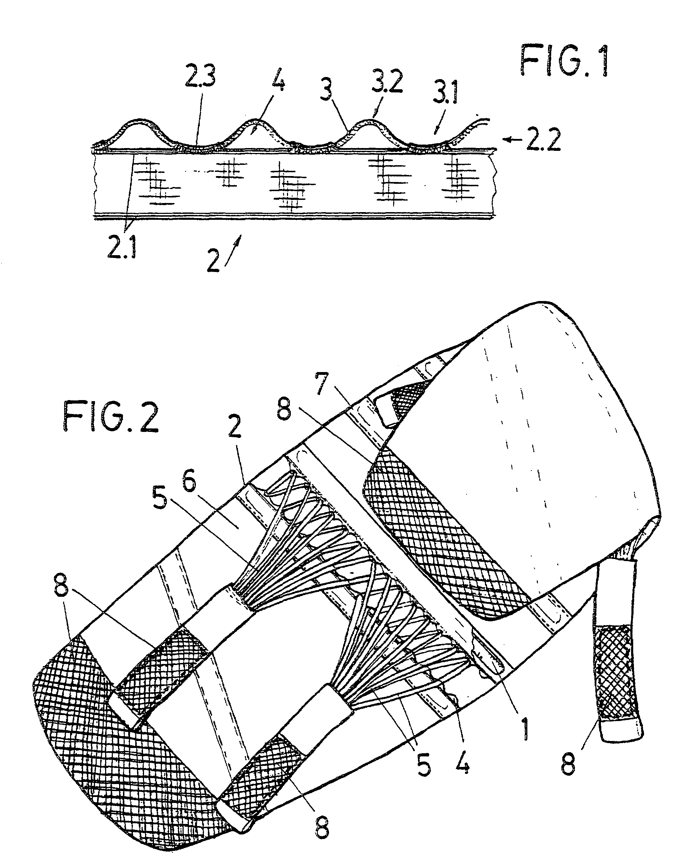

Figure 1 shows the band of the invention in one of its segments, sectioned in order to show its construction more clearly.

Figure 2 shows this band incorporated in an example of an embodiment as a corset which is described below, open and ready to be used.

### PREFERRED EMBODIMENT OF THE INVENTION

In view of the above, the present invention relates to a band with eyelets and housing for stays, from among those bands which are woven or not, used to house stays (1) used in clothing and orthopaedic items characterised by a double band (2) with lateral seams (2.1) and provided on one of its sides (2.2) with a lace (3) resistant to tension, connected with even or different spacing to band (2) at points (3.1) provided with suitable means, at which junctions half wave segments (3.2) of lace (3) are formed.

Said half wave segments (3.2) of lace (3), set at even or different separations, are used as eyelets (4) for tightening and closing laces (4) of the item.

For a corset added to said bands (2) with eyelets (4) are fabric portions (6) sewn intermediately which may or may not incorporate further conventional bands (7) with more stays (1), as well as external segments provided with conventional closing means, such as self-adhering textile items (8).

This description is not continued in the understanding that any expert in the field would have sufficient information to comprehend the scope of the invention and the advantages derived thereof, as well as to be capable of reproducing it.

It is also understood that as long as the essence of the invention remains unaltered any variations in materials, shape, size and arrangement of the elements may vary within the same characterisation.

The terms employed in the description and its meaning must always be understood in a non-limiting manner.

## Claims

1. Band with an eyelet and housing for stays from among woven or other bands, which house stays (1) used in clothing and orthopaedic items, essentially **characterised in that** it consists of a double band (2) with lateral seams (2.1) and provided on one side (2.2) with a tension resistant lace (3), connected at even or different spacing to band (2) at points (3.1) provided with suitable means, connections with which half-wave shaped segments (3.2) are formed of lace (3).

2. Band with an eyelet and housing for stays as in previous claim, **characterised in that** the half-wave segments (3.2) of lace (3) placed at even or different spacing from each other are used as eyelets (4) of laces (5) for tightening and closing of the item.

3. Band with an eyelet and housing for stays as in previous claims, **characterised in that** added to the aforementioned bands (2) with eyelets are fabric portions (6) woven intermediately, and which may or may not incorporate further conventional bands (7) with stays (1), as well as end segments incorporating self-adhering textile elements (8) or similar elements.

## Patentansprüche

1. Band mit einer Öse und einer Aufnahme für Korsettstangen aus Gewebestreifen und anderen Streifen, das Korsettstangen (1) aufnimmt, die in Bekleidung und orthopädischen Artikeln eingesetzt werden, grundsätzlich **dadurch gekennzeichnet, dass** es ein Doppelband (2) mit Seitennähten (2.1) umfasst und auf einer der Seiten (2.2) mit einem zugfesten Streifen (3) versehen ist (2.2), der an den Stellen (3.1) in gleichmässigen oder ungleichmässigen Abständen mit dem Band (2) verbunden ist und das geeignete Mittel oder Verbindungen aufweist, mit denen Abschnitte (3.2) des Streifens (3) in Halbwellenform gebildet werden.

2. Band mit einer Öse und einer Aufnahme für Korsettstangen gemäss dem vorhergehenden Patentanspruch, **dadurch gekennzeichnet, dass** der Abschnitt (3) des Streifens in Halbwellenform, der in gleichmässigen oder zueinander ungleichmässigen Abständen angeordnet ist, als Öse (4) der Streifen (5) benutzt wird, um den Artikel festzuziehen und zusammenzuhalten.

3. Band mit einer Öse und einer Aufnahme für Korsettstangen gemäss der vorhergehenden Patentansprüche, **dadurch gekennzeichnet, dass** ausser der vorher erwähnten Bänder (2) mit Ösen, dazwischenliegende Gewebeteile (6) vorhanden sind, die bei Bedarf weitere zusätzliche Bänder (7) mit Korsettstangen (1), sowie äussere Abschnitte aufnehmen können, die selbstklebende oder ähnliche textile Elemente (8) mit einschliessen.

## Revendications

1. Bande munie d'un oeillet et d'un logement pour baleines à bandes de tissu et à autres bandes contenant des baleines (1) utilisées sur des vêtements et des articles orthopédiques, caractérisée principalement par le fait qu'elle comprend une double bande (2) avec des coutures latérales (2.1) et munie sur l'une de ses faces (2.2) d'un lien (3) résistant à la tension, relié à la bande (2) par des séparations uniformes ou différentes en points (3.1) munie de moyens adéquats, connexions avec lesquelles l'on forme des segments (3.2) de lien (3) en forme de demi-vague.

2. Bande munie d'un oeillet et d'un logement pour baleines d'après la revendication précédente, **caractérisée par le fait que** le segment (3) de lien en forme de demi-vague placé dans des séparations uniformes ou différentes entre elles est utilisé comme oeillet (4) des liens (5) pour tendre et serrer l'article.

3. Bande munie d'un oeillet et d'un logement pour baleines d'après les revendications précédentes, **caractérisée par le fait que**, en plus des bandes (2) précédemment citées munies d'oeillets, il y a présence de portions (6) de tissu tissées de façon intermédiaire et pouvant comprendre ou non d'autres bandes supplémentaires (7) avec des baleines (1), ainsi que des segments aux extrémités comprenant des éléments (8) textiles autoadhésifs ou des éléments similaires.
